# EUROPÄISCHE PATENTSCHRIFT

(11) **EP 0 728 744 B1**
(45) Veröffentlichungstag und Bekanntmachung des Hinweises auf die Patenterteilung: **10.04.2002**
(21) Anmeldenummer: 96107208.9
(22) Anmeldetag: 31.05.1994
(51) Int. Cl.: C07D 213/81, C07D 215/48, C07D 215/50, C07D 241/24, C07B 43/06, C07D 213/79, C07D 213/803

(54) **Verfahren zur Herstellung von Carbonsäuren stickstoffhaltiger aromatischer Heterocyclen**
Process for the preparation of carboxylic acids of nitrogen-containing heterocycles
Procédé pour la préparation d'acides carboxyliques de composés hétérocycliques contenant de l'azote

(30) Priorität: 01.06.1993 CH 163093
(43) Veröffentlichungstag der Anmeldung: 28.08.1996
(62) Teilanmeldung aus: 94108389.1
(73) Patentinhaber: Lonza AG, 3930 Visp (CH)
(72) Erfinder: Roduit, Jean-Paul, Dr., Sierre (Kanton Wallis) (CH); Wellig, Alain, Ried-Mörel (Kanton Wallis) (CH); Amacker, Karin, Eischoll (Kanton Wallis) (CH); Eyer, Martin, Dr., Glis (Kanton Wallis) (CH)
(74) Vertreter: Weinhold, Peter, Dr.rer.nat. Dipl.-Chem.

(56) Entgegenhaltungen:
- EP-A- 0 068 219
- DE-A- 2 056 433
- DE-A- 2 539 435
- US-A- 2 780 624

## Beschreibung

Die Erfindung betrifft ein neues Verfahren zur Herstellung von Carbonsäuren stickstoffhaltiger aromatischer Heterocyclen (N-Heterocyclen) aus entsprechenden Carboxamiden durch alkalische Hydrolyse.

Sowohl die Carboxamide als auch die Carbonsäuren der N-Heterocyclen sind wichtige Zwischenprodukte zur Herstellung von Pharmazeutika.
Eine Carbamoylierungs-Methode von N-Heterocyclen ist aus der DE-PS 2 056 433 bekannt. Dieses Verfahren zeichnet sich dadurch aus, dass zur Bildung des Carbamoylradikals ein Redox-System aus R-OOH + Eisen (II) verwendet wird. R bedeutet hier Wasserstoff, Alkyl oder Cycloalkyl.
Eisen (II) muss dabei in stöchiometrischen Mengen oder sogar im Überschuss angewendet werden, was offensichtlich bei Synthesen im technischen Massstab grosse Abwasser- und Abfallprobleme mit sich bringt. Die verwendeten Alkylhydroperoxide sind teuer und zudem wegen ihrer Brisanz gefährlich zu handhaben.

Die alkalische Hydrolyse von Pyrazin-2,3-dicarboxamid unter Bildung von Pyrazin-2-carboxamid-3-carbonsäure wird in der US-A-2 780 624 beschrieben. Die EP-A-0 068 219 beschreibt die Herstellung von Pyridincarbonsäuren durch alkalische Hydrolyse von Pyridincarboxamiden.

Aufgrund dieser geschilderten Nachteile bestand die Aufgabe,ein Carbamoylierungsverfahren zu erarbeiten, das im technischen Massstab einsetzbar ist und ohne diese Mängel funktioniert.

Mit dem Verfahren gemäss Patentanspruch 1 konnte die Aufgabe auf überraschend einfache Weise gelöst werden.

Unter N-Heterocyclen werden zweckmässig Verbindungen aus der Reihe der Pyridine, Chinoline, Isochinoline, Pyrimidine, Pyridazine, Pyrazine, Chinoxaline, Chinazoline, Acridine oder der Benzimidazole verstanden.
Diese Verbindungen können gegebenenfalls mit einem oder mehreren Substituenten aus der

Reihe Alkyl, Alkoxy, Alkanoyl, Alkoxycarbonyl, Arylalkyl, Aryloxycarbonyl, Halogen, Carboxyl, Cyan, Amino, Alkylamino oder Dialkylamino versehen sein.

Die in den genannten Substituenten vorkommenden Alkylgruppen sind entweder linear oder verzweigt und haben zweckmässig 1 bis 6, bevorzugt 1 bis 4,C-Atome.

Unter einer Arylgruppe wird zweckmässig eine gegebenenfalls mit den genannten Substituenten substituierte Phenylgruppe verstanden. Halogen steht üblicherweise für Fluor, Chlor, Brom oder Iod.

Formamid wird zweckmässig in einer Menge von 3 bis 35 mol, bevorzugt in einer Menge von 5 bis 6 mol,pro mol eingesetztem N-Heterocyclus, zugesetzt.
Peroxodischwefelsäure oder das Peroxodisulfat wird von Vorteil ebenfalls in einem leichten Überschuss,d.h. in einer Menge von zweckmässig 1.1 bis 3.0 mol pro mol eingesetztem N-Heterocyclus,zudosiert.
Von Vorteil werden die Peroxodisulfate der Peroxodischwefelsäure vorgezogen. Geeignete Peroxodisulfate sind das leicht zugängliche Natrium-, Kalium- oder Ammoniumperoxodisulfat.

Zur Verbesserung der Selektivität der Carbamoylierung wird in Gegenwart einer starken Säure, bevorzugt in Gegenwart von Schwefelsäure, gearbeitet.
Die Zugabe eines Lösungsmittels ist nicht zwingend, da grundsätzlich das im Überschuss angewendete Formamid diese Funktion übernehmen kann.
Allerdings ist es möglich, in Gegenwart eines polaren inerten Lösungsmittels zu arbeiten. Als besonders geeignet hat sich Acetonitril herausgestellt.

Die Reaktionstemperatur liegt zweckmässig zwischen 20 und 80°C, bevorzugt zwischen 65 und 75°C.

Das resultierende Carboxamid kann bereits nach relativ kurzer Zeit nach beendeter Zugabe von Peroxodischwefelsäure oder Peroxodisulfat auf fachmännische Weise aus dem Reaktionsgemisch isoliert werden. In der Regel fällt das Carboxamid in guter Ausbeute von grösser 80% und hoher Reinheit an.

Abhängig von der Substitution der eingesetzten N-Heterocyclen können bei der erfindungsgemässen Umsetzung zwei Carboxamidfunktionen eingeführt werden. Dies ist insbesondere bei "elektronenschiebenden" Gruppen wie z. B. bei den Alkylgruppen der Fall. So resultiert bei der erfindungsgemässen Umsetzung des 4-Methylpyridins das 4-Methylpyridin-2,6-dicarboxamid.

Die resultierenden Carboxamide können entweder isoliert werden oder direkt alkalisch zu den entsprechenden Carbonsäuren hydrolysiert werden. Enthalten die erfindungsgemäss hergestellten Carboxamide CN-Gruppen als Substituenten, werden diese in der Regel ebenfalls zur Carbonsäure hydrolysiert. So resultiert bei der alkalischen Hydrolyse des aus 4-Cyanpyridin hergestellten 4-Cyan-2-pyridincarboxamids die als Zwischenprodukt für Pharmazeutika bedeutsame Pyridin-2,4-dicarbonsäure.

### Beispiel 1

### a) Verfahren zur Herstellung von 4-Cyan-2-pyridin-carboxamid

85 g (0.82 mol) 4-Cyanpyridin wurden bei Raumtemperatur in 700 ml Acetonitril vorgelegt. Dann wurden 32.4 g (0.32 mol) Schwefelsäure 98% zugegeben. Die resultierende weisse Suspension wurde auf 60°C erwärmt worauf 201.3 g (4.47 mol) Formamid in 52 g Wasser zugegeben wurde. Die erhaltene klare Lösung erhitzte man auf 70°C, worauf über einen Zeitraum von 2 Stunden 281.3 g (1.23 mol) Ammoniumperoxodisulfat portionenweise zudosiert wurde (Exothermie!). Nach beendeter Zugabe wurde bei 74°C während 75 Minuten weitergerührt, dann 880 ml Wasser zugegeben und unter Vakuum das Azeotrop Wasser / Acetonitril abdestilliert. Die weiss-gelbe Suspension wurde dann bei 80°C filtriert, der Filterkuchen mit 80°C warmem Wasser gewaschen und unter Vakuum getrocknet.
Man erhielt 117 g (87.4%) des Titelproduktes mit einem Gehalt von ca. 90% (HPLC).

### b) Verfahren zur Herstellung von Pyridin-2,4-dicarbonsäure

80 g (0.5 mol) 4-Cyan-2-pyridincarboxamid wurden in 155 ml Wasser aufgeschlammt. Bei 80°C wurden dann 170.3g 30%ige Natronlauge während 30 Minuten zugetropft, worauf eine gelbe Lösung entstand. Nach 30 Minuten Rühren wurde mit konzentrierter Salzsäure auf pH 1.5 gebracht, die resultierende weisse Suspension wurde gekühlt, filtriert und der Filterkuchen mit Wasser gewaschen. Danach wurde der Filterkuchen nochmals in Wasser aufgeschlämmt, mit Salzsäure auf pH 1 gestellt und bei 95°C gelöst. Beim darauffolgenden Abkühlenlassen kristallisierte die Pyridin-2,4-dicarbonsäure als Monohydrat. Nach dem Trocknen im Vakuum bei 115°C erhielt man 75 g (83.1%) des wasserfreien Titelproduktes mit einem Gehalt von grösser als 97% (HPLC).

Die nachfolgenden Beispiele wurden analog zu Beispiel 1a durchgeführt

| Beispiel | Ausgangsprodukt | Produkt | Ausbeute |
|---|---|---|---|
| 2 | Pyridin-2,3-dicarbonsäure | 5-Carbamoyl-pyridin-2,3-dicarbonsäure | 70% |
| | | | |
| 3 | 4-Methylpyridin | 4-Methylpyridin-2,6-dicarboxamid | 81% |
| | | | |
| 4 | 4-Chlorpyridin | 4-Chlorpyridin-2-carboxamid | 55% |
| | | | |
| 5 | 2-Methyl-5-ethylpyridin | 6-Methyl-3-ethylpyridin-2-carboxamid | 40% |
| | | | |
| 6 | 2,5-Dimethylpyrazin | 3,6-Dimethyl-2,5-pyrazindicarboxamid | 15% |
| | | | |
| 7 | 2-Methylchinolin | 2-Methyl-4-chinolincarboxamid | 90% |
| | | | |
| 8 | 6-Methyl-2-pyridincarbonitril | 6-Cyan-2-methyl-3-pyridincarboxamid | 25% |
| | | | |
| | | | |
| 9 | 6-Chlor-2-pyridincarbonitril | 6-Cyan-2-chlor-3-pyridincarboxamid | 30% |

### Beispiel 10

### Verfahren zur Herstellung von Pyridin-2,4-dicarbonsäure aus Isonikotinsäure

80 g (0.65 mol) Isonikotinsäure wurden bei Raumtemperatur in 600 ml Acetonitril suspendiert. Dann wurden 26 g (0.26 mol) Schwefelsäure 98%, 161 g (3.58 mol) Formamid und 42.4 g Wasser zugegeben.
Die Suspension wurde auf 70°C erwärmt, worauf portionenweise 208.7 g (0.91 mol) Ammoniumperoxodisulfat so zugegeben wurde, dass die Temperatur 75°C nicht überstieg. Nach Rühren während 90 Minuten bei 73°C wurden 650 ml Wasser hinzugefügt. Darauf wurde die Suspension filtriert und der Filterkuchen mit 150 ml Wasser gewaschen.
Die erhaltene 2-Carbamoyl-pyridin-4-carbonsäure wurde darauf in 350 ml Wasser suspendiert und auf 80°C erwärmt. Dann wurden 208 g Natronlauge 30% (1.56 mol) während 15 Minuten zugegeben. Es wurde darauf nachgerührt, bis keine NH₃-Entwicklung mehr zu beobachten war. Das restliche NH₃ wurde durch Erwärmen der Lösung auf 95°C abgetrennt.

Durch vorsichtiges Ansäuern der Lösung mit konz. HCI auf pH 1 und anschliessendes Abkühlen kristallisierte das Titelprodukt aus. Bei 5°C wurde die Suspension filtriert. Das resultierende Produkt wurde darauf durch erneutes Aufnehmen in 800 ml siedendem Wasser und durch anschliessendes Ansäuern mit konz. HCl auf pH 1 in Lösung gebracht und durch anschliessendes Abkühlen rekristallisiert. Nach Filtration, Waschen des Filtergutes mit Wasser und Trocknen im Vakuum konnten 81.5 g (75%) des Titelproduktes mit einem Gehalt nach HPLC von >98% erhalten werden.

## Patentansprüche

1. Verfahren zur Herstellung von Carbonsäuren von stickstoffhaltigen aromatischen Heterocyclen, bei welchem eine stickstoffhaltige aromatische heterocyclische Verbindung mit Formamid in Gegenwart einer starken Säure umgesetzt und das erhaltene Carboxamid anschließend durch alkalische Hydrolyse in die Carbonsäure umgewandelt wird,
**dadurch gekennzeichnet, dass** die Umsetzung der stickstoffhaltigen aromatischen heterocyclischen Verbindung zum Carboxamid unter zusätzlicher Anwesenheit von Peroxodischwefelsäure oder einem Peroxodisulfat erfolgt.

2. Verfahren nach Patentanspruch 1, **dadurch gekennzeichnet, dass** in Gegenwart von einem Peroxodisulfat gearbeitet wird.

3. Verfahren nach Patentanspruch 2, **dadurch gekennzeichnet, dass** Ammoniumperoxodisulfat eingesetzt wird.

4. Verfahren nach einem der Patentansprüche 1 bis 3, **dadurch gekennzeichnet, dass** die Peroxodischwefelsäure oder das Peroxodisulfat in einer Menge von 1.1 bis 3.0 mol pro mol des eingesetzten N-Heterocyclus eingesetzt wird.

5. Verfahren nach einem der Patentansprüche 1 bis 4, **dadurch gekennzeichnet, dass** das Formamid in einer Menge von 3 bis 35 mol pro mol des eingesetzten N-Heterocyclus eingesetzt wird.

6. Verfahren nach einem der Patentansprüche 1 bis 5, **dadurch gekennzeichnet, dass** als starke Säure Schwefelsäure eingesetzt wird.

7. Verfahren nach einem der Patentansprüche 1 bis 6, **dadurch gekennzeichnet, dass** die Umsetzung bei einer Temperatur zwischen 20 und 80°C durchgeführt wird.

8. Verfahren gemäß einem der Patentansprüche 1 bis 7, **dadurch gekennzeichnet, dass** das Carboxamid vor der alkalischen Hydrolyse isoliert wird.

9. Verfahren gemäß einem der Patentansprüche 1 bis 7, **dadurch gekennzeichnet, dass** es ohne Isolierung des Carboxamids durchgeführt wird.

10. Verfahren gemäß einem der Patentansprüche 1 bis 9 zur Herstellung von Pyridin-2,4-dicarbonsäure aus 4-Cyanpyridin oder Isonikotinsäure.

## Claims

1. Process for the production of carboxylic acids of nitrogenous aromatic heterocycles, in which a nitrogenous aromatic heterocyclic compound is reacted with formamide in the presence of a strong acid and the resultant carboxamide is then converted into the carboxylic acid by alkaline hydrolysis, **characterised in that** the reaction of the nitrogenous aromatic heterocyclic compound to yield the carboxamide proceeds in the additional presence of peroxydisulfuric acid or a peroxydisulfate.

2. Process according to claim 1, **characterised in that** the process is performed in the presence of a peroxydisulfate.

3. Process according to claim 2, **characterised in that** ammonium peroxydisulfate is used.

4. Process according to one of claims 1 to 3, **characterised in that** the peroxydisulfuric acid or the peroxydisulfate is introduced in a quantity of 1.1 to 3.0 mol per mol of introduced N-heterocycle.

5. Process according to one of claims 1 to 4, **characterised in that** the formamide is introduced in a quantity of 3 to 35 mol per mol of introduced N-heterocycle.

6. Process according to one of claims 1 to 5, **characterised in that** sulfuric acid is used as the strong acid.

7. Process according to one of claims 1 to 6, **characterised in that** the reaction is performed at a temperature of between 20 and 80°C.

8. Process according to one of claims 1 to 7, **characterised in that** the carboxamide is isolated before the alkaline hydrolysis.

9. Process according to one of claims 1 to 7, **characterised in that** it is performed without isolating the carboxamide.

10. Process according to one of claims 1 to 9 for the production of 2,4-pyridinedicarboxylic acid from 4-cyanopyridine or isonicotinic acid.

## Revendications

1. Procédé pour la préparation d'acides carboxyliques de composés hétérocycliques aromatiques contenant de l'azote, dans lequel une combinaison, hétérocyclique aromatique contenant de l'azote, avec le formiamide est transformée en présence d'un acide fort et le carboxamide obtenu est ensuite converti par hydrolyse alcaline en acide carboxylique,
**caractérisé en ce que** la transformation en carboxamide de la combinaison hétérocyclique aromatique contenant de l'azote a lieu en présence d'un ajout d'acide peroxodisulfurique ou d'un peroxodisulfate.

2. Procédé selon la revendication 1 **caractérisé en ce qu'**on opère en présence d'un peroxodisulfate.

3. Procédé selon la revendication 2 **caractérisé en ce qu'**on utilise du peroxodisulfate d'ammonium.

4. Procédé selon l'une des revendications 1 à 3 **caractérisé en ce qu'**on utilise l'acide peroxodisulfurique ou le peroxodisulfate selon un rapport molaire de 1,1 à 3,0 par mole du n-hétérocyclique utilisé.

5. Procédé selon l'une des revendications 1 à 4 **caractérisé en ce qu'**on utilise le formiamide selon un rapport molaire de 3 à 35 par mole du n-hétérocyclique utilisé.

6. Procédé selon l'une des revendications 1 à 5 **caractérisé en ce qu'**on utilise comme acide fort de l'acide sulfurique.

7. Procédé selon l'une des revendications 1 à 6 **caractérisé en ce qu'**on réalise la transformation à une température comprise entre 20 et 80 °C.

8. Procédé selon l'une des revendications 1 à 7 **caractérisé en ce qu'**on isole le carboxamide avant l'hydrolyse alcaline.

9. Procédé selon l'une des revendications 1 à 7 **caractérisé en ce qu'**il est réalisé sans isolement du carboxamide.

10. Procédé selon l'une des revendications 1 à 9 pour fabriquer de l'acide pyridine-2,4-dicarboxylique à partir de 4-cyanopyridine ou d'acide isonicotinique.
